# EUROPEAN PATENT APPLICATION

(11) **EP 3 951 390 A1**
(43) Date of publication of application: **09.02.2022**
(21) Application number: 20777335.9
(22) Date of filing: 19.03.2020
(51) Int. Cl.: G01N 33/543, G01N 33/569

(54) **IMMUNOCHROMATOGRAPHIC ANALYSIS METHOD AND TEST STRIP FOR USE IN SAID METHOD**

(30) Priority: 25.03.2019 JP 2019055974
(71) Applicant: Sekisui Medical Co., Ltd., Tokyo 103-0027 (JP)
(72) Inventor: AZUMA Kanako, Tokyo 103-0027 (JP); YAJI Shohei, Tokyo 103-0027 (JP); MORITA Motoki, Tokyo 103-0027 (JP)
(74) Representative: Hoffmann Eitle
(86) International application number: PCT/JP2020/012387
(87) International publication number: WO 2020/196298

(57) **Abstract**

The invention aims to provide an immunochromatographic assay method in which the non-specific reaction is suppressed, and a test strip to be used in the method. The object can be achieved by an immunochromatographic assay method for an analyte, the method including using a test strip including the following (1) to (3): (1) a sample pad containing a sample application region for applying a biological sample that may contain the analyte; (2) an insoluble membrane containing at least one detection region to which a binding partner immunologically reactive with the analyte is immobilized; and (3) a conjugate containing a binding partner immunologically reactive with the analyte, the binding partner being immobilized to a label; characterized in that the conjugate is retained in a conjugate immobilization region together with an anionic surfactant.

## Description

### TECHNICAL FIELD

The present invention relates to an immunochromatographic assay method for detecting an analyte such as influenza virus, and a test strip for use in the method. The invention also relates to a method of suppressing non-specific reaction in an immunochromatographic assay method for detecting an analyte such as influenza virus.

### BACKGROUND ART

For the purpose of early diagnosis of a disease, POCT (point-of-care testing) is carried out in real time at the location of a patient in order to quickly obtain information useful for the diagnosis and the treatment. In POCT, immunoassays for detecting analytes that cause diseases are widely carried out using binding partners specifically reactive with the analytes. Among such immunoassays, immunochromatography is especially widely used because of the simplicity of its operation. However, such an immunoassay tends to be accompanied by non-specific reaction in cases where the specimen contains contaminants such as bacteria, viruses, and proteins with cross-antigenicity. This is problematic since such false-positive reactions may prevent accurate diagnosis.

A variety of attempts have been made in order to suppress non-specific reaction due to the contaminants in the samples. For example, Patent Document 1 discloses a reagent composition for immunochromatography, the reagent composition comprising a nonionic surfactant, N,N-bis(2-hydroxyethyl)glycine buffer, and casein. However, the suppression of non-specific reaction has been demonstrated only for cases where these substances were used in combination. Therefore, a technique capable of effectively suppressing the non-specific reaction due to the contaminants in the samples even by the addition of a single substance has been demanded.

### CITATION LIST

### PATENT LITERATURE

Patent Document 1: JP-A-2012-37253

### SUMMARY OF INVENTION

### TECHNICAL PROBLEM

An object of the invention is to provide an immunochromatographic assay method in which the non-specific reaction is suppressed, and a test strip to be used in the method.

### SOLUTION TO PROBLEM

The present inventors discovered that, in an immunochromatographic assay method for an analyte, effective suppression of the non-specific reaction is possible when the method has the following configuration, thereby completing the invention.

More specifically, the invention is as follows.
<1> An immunochromatographic assay method for an analyte, the method comprising using a test strip comprising following (1) to (3):
   (1) a sample pad containing a sample application region for applying a biological sample that may contain the analyte;
   (2) an insoluble membrane containing at least one detection region to which a binding partner immunologically reactive with the analyte is immobilized; and
   (3) a conjugate containing a binding partner immunologically reactive with the analyte, the binding partner being immobilized to a label;
      characterized in that the conjugate is retained in a conjugate immobilization region together with an anionic surfactant.
<2> The immunochromatographic assay method according to <1>, wherein the analyte is a respiratory tract virus.
<3> The immunochromatographic assay method according to <1> or <2>, wherein the binding partner is a monoclonal antibody.
<4> The immunochromatographic assay method according to any one of <1> to <3>, characterized in that the conjugate immobilization region is provided, as a conjugate region between the sample application region and the detection region in the (1) or (2).
<5> The immunochromatographic assay method according to <4>, wherein a concentration of the anionic surfactant is 0.1 to 10% by mass in terms of a concentration in a conjugate application solution for application to the conjugate region in preparation of the test strip.
<6> The immunochromatographic assay method according to any one of <1> to <5>, wherein the biological sample is respiratory secretions.
<7> The immunochromatographic assay method according to any one of <1> to <6>, wherein the respiratory tract virus is influenza B virus.
<8> The immunochromatographic assay method according to any one of <1> to <7>, wherein the anionic surfactant is a sodium polyoxyethylene alkyl ether sulfate-based anionic surfactant.
<9> An immunochromatographic test strip comprising following (1) to (3):
   (1) a sample pad containing a sample application region for applying a biological sample that may contain an analyte;
   (2) an insoluble membrane containing at least one detection region to which a binding partner immunologically reactive with the analyte is immobilized; and
   (3) a conjugate immobilization region containing a conjugate together with an anionic surfactant.
<10> The immunochromatographic test strip according to <9>, wherein the analyte is a respiratory tract virus.
<11> The immunochromatographic test strip according to <9> or <10>, wherein the binding partner is a monoclonal antibody.
<12> The immunochromatographic test strip according to any one of <9> to <11>, characterized in that the conjugate immobilization region is provided, as a conjugate region between the sample application region and the detection region in the (1) or (2).
<13> The immunochromatographic test strip according to any one of <9> to <12>, wherein a concentration of the anionic surfactant is 0.1 to 10% by mass in terms of a concentration in a conjugate application solution for application to the conjugate region in preparation of the test strip.
<14> The immunochromatographic test strip according to any one of <9> to <13>, wherein the biological sample is respiratory secretions.
<15> The immunochromatographic test strip according to any one of <9> to <14>, wherein the respiratory tract virus is influenza B virus.
<16> The immunochromatographic test strip according to any one of <9> to <15>, wherein the anionic surfactant is a sodium polyoxyethylene alkyl ether sulfate-based anionic surfactant.
<17> A method of suppressing non-specific reaction in an immunochromatographic assay method for an analyte, the method comprising using a test strip comprising following (1) to (3) :
   (1) a sample pad containing a sample application region for applying a biological sample that may contain the analyte;
   (2) an insoluble membrane containing at least one detection region to which a binding partner immunologically reactive with the analyte is immobilized; and
   (3) a conjugate immobilization region containing a conjugate together with an anionic surfactant.
<18> The method of suppressing non-specific reaction according to <17>, wherein the analyte is a respiratory tract virus.
<19> The method of suppressing non-specific reaction according to <17> or <18>, wherein a concentration of the anionic surfactant is 0.1 to 10% by mass in terms of a concentration in a conjugate application solution for application to a conjugate region in preparation of the test strip.
<20> The method of suppressing non-specific reaction according to any one of <17> to <19>, wherein the biological sample is respiratory secretions.
<21> The method of suppressing non-specific reaction according to any one of <17> to <20>, wherein the respiratory tract virus is influenza B virus.
<22> The method of suppressing non-specific reaction according to any one of <17> to <21>, wherein the anionic surfactant is a sodium polyoxyethylene alkyl ether sulfate-based anionic surfactant.

### ADVANTAGEOUS EFFECTS OF INVENTION

By the invention, non-specific reaction in an immunochromatographic assay for an analyte can be effectively suppressed.

### BRIEF DESCRIPTION OF DRAWINGS

[Fig. 1] Fig. 1 is a schematic diagram of a test strip used in one embodiment of the invention.
[Fig. 2] Fig. 2 is a schematic diagram of a test strip used in one embodiment of the invention.

### DESCRIPTION OF EMBODIMENTS

### (Analyte)

In the invention, examples of the analyte include viruses, and physiologically active substances such as proteins that can be generally measured using antigen-antibody reaction. Examples of the viruses include influenza viruses such as influenza A virus and influenza B virus, hepatitis B virus, hepatitis C virus, and human immunodeficiency virus. Examples of the proteins include human hemoglobin, hepatitis B virus antibodies, hepatitis C virus antibodies, and human immunodeficiency virus antibodies.

The analyte in the invention is preferably a respiratory tract virus. The respiratory tract virus in the present description is a virus that causes respiratory disease. The respiratory tract virus in the invention is not limited as long as it is a virus that can be detected using antigen-antibody reaction, and examples of the respiratory tract virus include coronaviruses such as coronavirus 229E, coronavirus OC43, and coronavirus NL63; influenza viruses such as influenza A virus, influenza B virus, influenza C virus, parainfluenza virus 1, parainfluenza virus 2, and parainfluenza virus 3; RS viruses such as RS virus A and RS virus B; an adenovirus; rhinovirus; metapneumovirus; cytomegalovirus; and bocavirus. In particular, the analyte is preferably an influenza virus, more preferably influenza B virus. Most preferably, the later-described detection region is formed at a plurality of positions (for example, two positions) to detect influenza A virus and influenza B virus.

In the description, the "respiratory tract" is a general term for the organs involved in the respiration, and means the organs positioned from the nasal vestibule to the alveoli (lung), including the nasal cavity, pharynx, larynx, trachea, bronchi, and bronchioli.

### (Biological Sample)

Examples of the biological sample that may contain an analyte, used in the invention include substances mainly derived from the living body, such as body fluids; and extracts prepared by extraction of the analyte from these substances. Specific examples of the substances derived from the living body include blood, urine, stool, and respiratory secretions. In cases where the analyte is a respiratory tract virus, the biological sample is preferably respiratory secretions, such as nasal discharge derived from the nares, nasal cavity, pharynx, nasopharynx, or the like; sputum; or saliva. The living body-derived substances and the extracts thereof may be used as they are as samples, or may be appropriately diluted with a sample diluent to provide samples. They may also be appropriately filtered to provide samples.

### (Anionic Surfactant)

In the description, the anionic surfactant means a surfactant containing a hydrophobic group which is negatively ionized when the surfactant is dissolved in water. In the description, surfactants which are not ionized when the surfactants are dissolved in water are referred to as nonionic surfactants; surfactants containing a hydrophobic group which is positively ionized when the surfactants are dissolved in water are referred to as cationic surfactants; and surfactants which exhibit properties of anionic surfactants in the alkaline range, but which exhibit properties of cationic surfactants in the acidic range, are referred to as amphoteric surfactants.

As the anionic surfactant, a sulfonate-based anionic surfactant, a carboxylate-based anionic surfactant, a sodium polyoxyethylene alkyl ether sulfate-based anionic surfactant, or the like may be used. More specifically, examples of the sulfonate-based anionic surfactant include sodium β-naphthalene sulfonate-formaldehyde condensate (trade name: DEMOL NL); examples of the carboxylate-based anionic surfactant include special polycarboxylate-type polymer surfactant (trade name: DEMOL EP); and examples of the sodium polyoxyethylene alkyl ether sulfate-based anionic surfactant include sodium polyoxyethylene lauryl ether sulfate (trade name: EMAL 20C) and sodium polyoxyethylene alkyl ether sulfate (trade name: EMAL 20CM) . These anionic surfactants may be used individually, or two or more thereof may be used in combination. The anionic surfactant is preferably a sodium polyoxyethylene alkyl ether sulfate-based anionic surfactant.

As long as the effect of the invention is not deteriorated, other kinds of surfactants may be used in addition to the anionic surfactant. The other kinds of surfactants are preferably not used.

In the description, the "sulfonate-based anionic surfactant" means a surfactant which is a salt of a substance containing a sulfonate group (-SO₃H); the "carboxylate-based anionic surfactant" means an anionic surfactant which is a salt of a substance containing a carboxylate group (-COOH); and the "sodium polyoxyethylene alkyl ether sulfate-based anionic surfactant" means an anionic surfactant having a chemical structure of sodium polyoxyethylene alkyl ether sulfate.

The concentration of the anionic surfactant may be appropriately adjusted depending on the types of the anionic surfactant and the analyte. For example, from the viewpoint of the effect for suppressing the non-specific reaction, the concentration of the anionic surfactant in the conjugate application solution for application to the conjugate region in preparation of the test strip is 0.001 to 20% by mass, preferably 0.01 to 20% by mass, more preferably 0.05 to 20% by mass, still more preferably 0.1 to 20% by mass, most preferably 0.5 to 20% by mass. The upper limit of the concentration may be 10% by mass, 8% by mass, 5% by mass, 3% by mass, or 2% by mass.

### (Conjugate)

The conjugate contains an antibody immunologically reactive with the respiratory tract virus in which the antibody is immobilized to a label. In the description, a portion where the conjugate is immobilized in a sample pad, an insoluble membrane, a conjugate pad, a filter, or the like is referred to as a conjugate immobilization region. Regarding the mode of presence of the conjugate immobilization region to which the conjugate is immobilized, the conjugate immobilization region may be present as a conjugate pad in the state impregnated in a pad, provided separately from a sample pad, a third pad, and an insoluble membrane (type A), may be present as a conjugate region in a part of a sample pad or a part of an insoluble membrane (type B), or may be present alone as a conjugate reagent which is to be mixed with the specimen (type C) .

As the label used in the invention, a known label conventionally used for detection of a respiratory tract virus may be used. For example, the label is preferably colloidal metal particles such as colloidal gold particles or colloidal platinum particles; colored latex particles; magnetic particles; or the like. The label is especially preferably colloidal gold particles.

In the case of detecting influenza virus, the conjugate is preferably a conjugate in which an anti-influenza virus monoclonal antibody is immobilized on colloidal gold particles.

The following describes a test strip in which the mode of presence of the conjugate immobilization region is the type A.

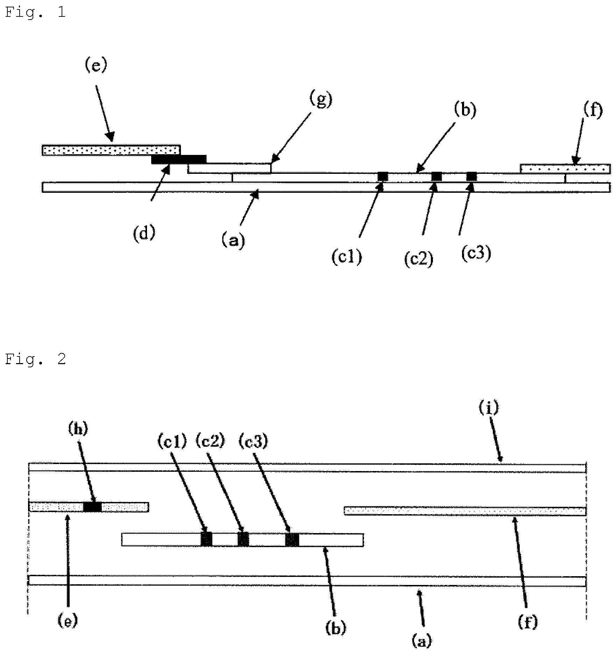

From upstream to downstream of the direction of the sample flow, a sample pad, a conjugate pad, a third pad, and an insoluble membrane are placed in this order. Each layer is placed such that at least part of the layer overlaps with the upstream/downstream layer(s). Fig. 1 illustrates an example of a test strip having such a configuration.

When a sample containing a respiratory tract virus is applied to the sample pad of the test strip, the respiratory tract virus flows through the sample pad to the conjugate pad located downstream. The conjugate pad contains an anionic surfactant, and, in the presence of the anionic surfactant, the respiratory tract virus contacts the conjugate, and passes through the pad while forming a first complex (aggregate). Thereafter, the first complex passes through a porous third pad placed in contact with the lower side of the conjugate pad, followed by development into the insoluble membrane.

On the part of the insoluble membrane, an antibody immunologically reactive with the respiratory tract virus is immobilized. Here, immune reaction occurs to cause the first complex to bind to the antibody immunologically reactive with the respiratory tract virus, resulting in the formation of an immobilized second complex. The immobilized second complex is then detected by means that detects a signal such as the absorbance, reflected light, fluorescence, or magnetism derived from the conjugate.

The following describes a test strip in which the mode of presence of the conjugate immobilization region is type B.

The difference from the test strip of type A is that the sample pad or the insoluble membrane is integrated with the conjugate pad. Fig. 2 illustrates an example of a test strip having a configuration in which part of a sample pad or an insoluble membrane is composed of a sample application region and a conjugate region.

The sample application region is a region where the sample containing the respiratory tract virus is applied, and the conjugate region is a region containing the conjugate. The sample application region is located upstream of the conjugate region.

The conjugate region is formed on the sample pad or an insoluble membrane such that the conjugate region is located downstream of the sample application region. The conjugate region contains an anionic surfactant. The conjugate region is preferably placed to form a line shape in the direction perpendicular to the direction of sample development, that is, perpendicular to the line connecting the center of the sample application region in the sample pad and the center of the downstream end of the later-mentioned insoluble membrane. In other words, the line-shaped conjugate region is preferably placed to form a line shape in the direction perpendicular to the longitudinal direction of the sample pad and the insoluble membrane. Although the conjugate region may be present in the insoluble membrane such that the conjugate region is located upstream of the detection region, it is preferably formed in the sample pad. In this case, the downstream side of the conjugate region in the sample pad does not necessarily need to be entirely occupied by the conjugate region, and the downstream side of the conjugate region may have a porous material portion which does not contain the conjugate.

The position of the conjugate region can be appropriately adjusted to an appropriate length by those skilled in the art. The centerline of the line of the line-shaped conjugate region is preferably positioned at least 3 mm upstream from the downstream end of the sample pad. In the description, the "centerline" of the conjugate region or the detection region means the centerline drawn in the direction perpendicular to the longitudinal direction of the sample pad, and does not mean the centerline drawn in the direction parallel to the longitudinal direction of the sample pad.

The sample containing the respiratory tract virus applied to the sample application region migrates from upstream to downstream. In the conjugate region, the respiratory tract virus and the conjugate form a first complex in the presence of the anionic surfactant. The first complex migrates further downstream. The first complex then forms a second complex with an antibody immobilized to the detection region, which antibody is immunologically reactive with the respiratory tract virus. The second complex immobilized to the detection region is then detected by means that detects a signal such as the absorbance, reflected light, fluorescence, or magnetism derived from the conjugate.

The following describes a test strip in which the mode of presence of the conjugate immobilization region is type C.

The difference from the test strip of type A is that there is no conjugate pad, and that the conjugate is present alone as a conjugate reagent. For example, a filter tip having a filter containing the conjugate therein is employed. This filter tip also contains an anionic surfactant. A sample diluent and a respiratory tract virus are passed through the filter tip to bind the conjugate to the respiratory tract virus, thereby forming a first complex (aggregate). By applying the resulting first complex to a test strip which is the same as type A except for the absence of the conjugate pad, a second complex can be formed in the detection region to which an antibody immunologically reactive with the respiratory tract virus is immobilized. The immobilized second complex is then detected by means that detects a signal such as the absorbance, reflected light, fluorescence, or magnetism derived from the conjugate.

### (Antibody Immunologically Reactive with Respiratory tract virus)

The antibodies immunologically reactive with the respiratory tract virus, used in the invention are antibodies capable of binding to the respiratory tract virus. The antibodies immunologically reactive with the respiratory tract virus are immobilized to the label and the detection region. Although the antibodies immobilized to the label and the detection region may be the same, different antibodies are preferably used for the label and the detection region. By using different antibodies as the antibody immobilized to the label and the antibody immobilized to the detection region, the competition between the reaction between the respiratory tract virus bound to the conjugate and the antibody in the detection region, and the reaction between the respiratory tract virus bound to the conjugate and the unreacted conjugate, can be suppressed. Further, the reactivity between the respiratory tract virus bound to the conjugate and the antibody in the detection region can be increased. As a result, the immunochromatographic test strip can have a good sensitivity. The different antibodies means that the types of the antibodies are different, that is, the antibodies recognize different epitopes.

The antibodies immobilized to the label and the detection region are preferably monoclonal antibodies. By the use of monoclonal antibodies, the specificity of the reaction can be increased.

Other than the whole molecules of these antibodies, functional fragments of the antibodies having antigen-antibody reaction activity are similarly regarded as the antibodies in the invention. Examples of the functional fragments of the antibodies include those obtained through the process of immunization of an animal, those obtained using genetic recombination techniques, and chimeric antibodies. Examples of the functional fragments of the antibodies include F(ab')₂ and Fab'. These functional fragments can be produced by treating the antibodies with a protease (such as pepsin or papain).

### (Sample Pad)

The sample pad is layered on the conjugate pad or the later-mentioned insoluble membrane such that the lower side of the downstream end of the sample pad is in contact with the upper side of the conjugate pad or the insoluble membrane. Examples of the porous material constituting the sample pad include pads made of non-woven fibers such as paper, cellulose mixture, nitrocellulose, polyester, acrylonitrile copolymer, glass, and rayon. The pad is especially preferably made of glass fibers (a glass fiber pad).

### (Third Pad)

In a test strip of the type A, for the purpose of removing non-specific reacting substances in the biological sample, the third pad is placed between the conjugate pad and the insoluble membrane. The third pad is preferably placed when necessary depending on the properties of the sample and/or the like. The third pad is not limited as long as the complex of the respiratory tract virus in the sample and the conjugate can pass through the third pad.

### (Insoluble Membrane)

The insoluble membrane that may be used in the invention contains at least one detection region to which an antibody immunologically reactive with the respiratory tract virus is immobilized. The antibody immunologically reactive with the respiratory tract virus can be immobilized to the insoluble membrane by a known method. In cases of a reagent for lateral flow immunochromatography, a solution containing the antibody at a predetermined concentration is prepared. Thereafter, the solution is applied to the insoluble membrane to form a line shape using, for example, an apparatus having a mechanism capable of traveling in a horizontal direction while discharging the solution from a nozzle at a constant rate. By drying the applied solution, the antibody can be immobilized.

The solution containing the antibody at a predetermined concentration can be prepared by adding the antibody to a buffer. Examples of the type of the buffer include conventionally used buffers such as phosphate buffer, tris buffer, and Good's buffer. The buffer has a pH within the range of preferably 6.0 to 9.5, more preferably 6.5 to 8.5, still more preferably 7.0 to 8.0. The buffer may also contain: salts such as sodium chloride; stabilizers such as sugars; preservatives; and antiseptics such as ProClin. Examples of the salts include not only those added for adjusting the ionic strength, such as sodium chloride, but also those added for the purpose of adjusting the pH of the buffer, such as sodium hydroxide.

After the immobilization of the antibody to the insoluble membrane, the portion other than the site of antibody immobilization may be blocked by coating with a solution or vapor of a conventionally used blocking agent.

Examples of the membrane constituting the insoluble membrane that may be used in the invention include known membranes that have been conventionally used as insoluble membranes for immunochromatographic test strips. Examples of the membrane include membranes constituted by fibers composed of polyethylene; polyethylene terephthalate; nylon; glass; a polysaccharide such as cellulose or cellulose derivative; or ceramic. Specific examples of the membrane include glass fiber filter papers and nitrocellulose membranes commercially available from Sartorius, Millipore, Toyo Roshi Kaisha, Ltd., Whatman, and the like.

The immunochromatographic test strip that may be used in the invention is preferably provided with an absorption pad at the downstream end of the insoluble membrane. The absorption pad is a portion having liquid absorbability, which portion controls the development of the sample by absorption of the sample that has migrated/passed through the insoluble membrane. As the absorption pad, a known absorption pad that has been conventionally used for immunochromatographic test strips may be used. For example, a filter paper may be used.

The non-specific reaction in the description means that a signal derived from the conjugate is detected, and hence that the biological sample is judged as positive, in spite of the fact that the respiratory tract virus as the analyte is absent or substantially absent in the biological sample.

### (Immunochromatographic Test Strip)

In the description, the "immunochromatography" means an immunoassay utilizing the nature of a porous material such as a cellulose membrane that allows a test specimen to flow slowly therethrough (capillary phenomenon) while dissolving a reagent. In the description, "using a test strip" means that the analyte contained in the biological sample is detected by bringing the test strip into contact with the biological sample. The immunochromatographic test strip is preferably placed on a solid support such as a plastic adhesive sheet. The solid support is constituted with a substance that does not prevent the capillary flow of the sample and the conjugate. The immunochromatographic test strip may be immobilized on the solid support using an adhesive or the like. In this case, the component of the adhesive or the like is also constituted with a substance that does not prevent the capillary flow of the sample and the conjugate. For the purpose of increasing the mechanical strength of the insoluble membrane, and preventing evaporation of water (drying) during the assay, the test strip may be laminated with a polyester film or the like. The immunochromatographic test strip may be used in a state where the test strip is stored or mounted in an appropriate container (housing) taking into account the size of the immunochromatographic test strip, the method of addition of the sample, the position where the sample is added, the position where each detection region is formed in the insoluble membrane, the detection method for the signal, and/or the like. The test strip stored or mounted in this state is referred to as "device".

In the description, the immunochromatographic assay method means an assay method utilizing immunochromatography.

### (Others)

When appropriate, the immunochromatographic test strip may be prepared with modification or alteration of the methods described in the Examples.

### EXAMPLES

The invention is described below concretely by way of Examples. However, these do not limit the scope of the invention. In the description, "%" represents "% by mass" unless otherwise specified.

### [Preparation of Anti-Influenza Virus Monoclonal Antibodies]

The anti-influenza A virus monoclonal antibodies and the anti-influenza B virus monoclonal antibodies used in the following tests were obtained by immunization of mice using recombinant influenza nucleoproteins as antigens, by a method conventionally used by those skilled in the art for production of monoclonal antibodies.

### [Test Strip Preparation Example 1] Preparation of Immunochromatographic Test Strip

### 1) Preparation of Colloidal Gold Particle-Labeled Anti-Influenza Virus Monoclonal Antibodies (Conjugates)

PBS containing an anti-influenza A virus monoclonal antibody at a concentration of 100 µg/mL and PBS containing an anti-influenza B virus monoclonal antibody at a concentration of 100 µg/mL were prepared. To 20 mL of 1 OD/mL colloidal gold particle solution, 1 mL of each antibody solution was added, and the resulting mixture was stirred at room temperature for 10 minutes. To each of the colloidal gold particle/anti-influenza A virus monoclonal antibody mixture and the gold colloid particle/anti-influenza B virus monoclonal antibody mixture, 2 mL of 10% aqueous bovine serum albumin (BSA) solution was added, and each resulting mixture was stirred for 5 minutes, followed by centrifugation at 10°C at 10,000 rpm for 45 minutes to obtain a precipitate (conjugate) . To each conjugate obtained, Conjugate Dilution Buffer (manufactured by Scripps) was added, and the conjugate was suspended therein.

### 2) Preparation of Sample Pad

Each conjugate prepared in 1) as described above was mixed with a solution containing 1.33% casein and 4% sucrose (pH 7.5) to a concentration of 8 to 20 OD/mL, to prepare a conjugate solution. Anionic surfactants were added to this conjugate solution at various concentrations. A glass fiber pad having a total length of 28 mm was soaked with the conjugate solution such that a line having a line width of 4 mm was formed. The pad was dried by heating at 70°C for 30 minutes, to provide a sample pad containing a conjugate region.

### 3) Preparation of Insoluble Membrane to Which Anti-Influenza Virus Monoclonal Antibody Is Immobilized (Antibody-Immobilized Membrane)

On a short-side end of a nitrocellulose membrane (pore size, 8 µm), an anti-influenza A virus monoclonal antibody, an anti-influenza B virus monoclonal antibody, and an anti-mouse IgG antibody, whose concentrations were adjusted to 0.75 mg/mL, 1.0 mg/mL, and 0.75 mg/mL, respectively, with phosphate buffer were applied to form lines in the direction perpendicular to the longitudinal direction of the insoluble membrane. Thereafter, the membrane was dried at 70°C for 45 minutes to provide an antibody-immobilized membrane. The lines were applied such that the anti-influenza A virus monoclonal antibody (c1), the anti-influenza B virus monoclonal antibody (c2), and the control antibody (c3) are arranged in this order from the upstream side when the test strip is assembled.

### 4) Preparation of Immunochromatographic Test Strip

The sample pad prepared in 2) was layered on the insoluble membrane obtained in 3), to prepare an immunochromatographic test strip.

### [Example 1]

Using the immunochromatographic test strip prepared in Test Strip Preparation Example 1, a detection test for influenza A and B viruses was carried out.

### 1. Test Method

(1) Samples
   Nasal mucus from a healthy subject, who was not infected with influenza, was mixed with a sample diluent, to provide each sample.
(2) Procedure
   After dispensing 135 µL of each sample prepared in (1) into a test tube, the immunochromatographic test strip was inserted into the tube, and the presence or absence of color development of the test lines A and B on the antibody-immobilized membrane in the detection regions was visually judged 5, 15, 30, and 45 minutes later.

### 2. Test Results

The results of the judgment of the presence/absence of color development of the test line B, which line indicates detection of influenza B virus, are shown in Tables 1 to 3.

**[Table 1]**

| Number | | 1 | 2 | 3 | 4 | 5 | 6 |
|---|---|---|---|---|---|---|---|
| Surfactant name | | EMULGEN 103 | EMULGEN 103 | AMIET 105 | AMIET 105 | FS30 | FS30 |
| Surfactant type | | Nonionic | Nonionic | Nonionic | Nonionic | Nonionic | Nonionic |
| Surfactant concentration (%) | | 5% | 1% | 5% | 1% | 5% | 1% |
| Specimen 1 | 5 | - | - | - | - | - | - |
| | 15 | - | - | - | - | - | - |
| | 30 | - | + | - | - | + | - |
| | 45 | - | + | - | + | + | + |
| Specimen 2 | 5 | - | - | - | - | - | - |
| | 15 | - | + | - | + | + | + |
| | 30 | + | + | + | + | + | + |
| | 45 | + | + | - | + | + | + |

**[Table 2]**

| Number | | 7 | 8 | 9 | 10 | 11 |
|---|---|---|---|---|---|---|
| Surfactant name | | EMAL 20C | EMAL 20CM | EMAL 20CM | AMPHITOL 20N | AMPHITOL 20N |
| Surfactant type | | Anionic | Anionic | Anionic | Amphoteric | Amphoteric |
| Surfactant concentration (%) | | 5% | 5% | 1% | 5% | 1% |
| Specimen 1 | 5 | - | - | - | - | - |
| | 15 | - | - | - | + | - |
| | 30 | - | - | - | + | + |
| | 45 | - | - | - | + | + |
| Specimen 2 | 5 | - | - | - | - | - |
| | 15 | - | - | - | - | + |
| | 30 | - | - | - | + | + |
| | 45 | - | - | - | + | + |

**[Table 3]**

| Number | | 12 | 13 | 14 | 15 | 16 |
|---|---|---|---|---|---|---|
| Surfactant name | | AMPHITOL 20Y | AMPHITOL 20Y | QUARTAMIN 24P | QUARTAMIN 24P | No addition |
| Surfactant type | | Amphoteric | Amphoteric | Cationic | Cationic | No addition |
| Surfactant concentration (%) | | 5% | 1% | 5% | 1% | - |
| Specimen 1 | 5 | - | - | + | - | - |
| | 15 | - | - | + | - | - |
| | 30 | - | - | + | + | - |
| | 45 | - | - | + | + | - |
| Specimen 2 | 5 | - | - | + | - | - |
| | 15 | - | + | + | - | - |
| | 30 | - | + | + | + | + |
| | 45 | + | + | + | + | + |

EMULGEN, AMIET, EMAL, AMPHITOL, and QUARTAMIN are registered trademarks.

In Tables 1 to 3, "-" represents the absence of color development of the test line B. The test line B indicates detection of influenza B virus. Thus, "-" indicates the absence of a false-positive result. "+" represents color development of the line, indicating the occurrence of a false positive result. None of the samples to which an anionic surfactant was added showed non-specific reaction in the detection of influenza B virus, but all of the samples to which a surfactant other than an anionic surfactant was added showed non-specific reaction in the detection of influenza B virus. None of the tested samples showed non-specific reaction in the detection of influenza A virus. It was thus shown that addition of an anionic surfactant to the sample diluent enables the prevention of non-specific reaction in the detection of influenza B virus.

### [Example 2]

1. Test Method
   A detection test for influenza A and B viruses was carried out in the same manner as in Example 1 except that different types and concentrations of surfactants were used.
2. Test Results
   The results of the judgment of the presence/absence of color development of the test line B, which indicates the detection of influenza B virus, are shown in Tables 4 and 5.

**[Table 4]**

| Number | | 17 | 18 | 19 | 20 |
|---|---|---|---|---|---|
| Surfactant name | | EMAL 20C | EMAL 20C | EMAL 20C | EMAL 20C |
| Surfactant type | | Anionic | Anionic | Anionic | Anionic |
| Surfactant concentration (%) | | 10% | 5% | 2.5% | 1% |
| Specimen 1 | 5 | - | - | - | - |
| | 15 | - | - | - | - |
| | 30 | - | - | - | - |
| | 45 | - | - | - | - |

**[Table 5]**

| Number | | 21 | 22 | 23 | 24 |
|---|---|---|---|---|---|
| Surfactant name | | EMAL 20CM | EMAL 20CM | EMAL 20CM | EMAL 20CM |
| Surfactant type | | Anionic | Anionic | Anionic | Anionic |
| Surfactant concentration (%) | | 10% | 5% | 2.5% | 1% |
| Specimen 1 | 5 | - | - | - | - |
| | 15 | - | - | - | - |
| | 30 | - | - | - | - |
| | 45 | - | - | - | - |

In Table 4 and Table 5, "-" represents the absence of color development of the test line B. The test line B indicates the detection of influenza B virus. Thus, "-" indicates the absence of a false-positive result. "+" represents color development of the line, indicating the occurrence of a false positive result. None of the tested samples showed non-specific reaction in the detection of influenza B virus.

It was thus found that an anionic surfactant is effective for suppressing non-specific reaction that occurs in the detection of influenza B virus even in cases where the concentration of the surfactant is low. None of the tested samples showed non-specific reaction in the detection of influenza A virus.

### INDUSTRIAL APPLICABILITY

By the invention, an immunochromatographic assay method in which the non-specific reaction is suppressed, and a test strip to be used in the method, can be provided.

### REFERENCE SIGNS LIST

- (a): Plastic adhesive sheet
- (b): Insoluble membrane
- (c1): Test line A
- (c2): Test line B
- (c3): Control line
- (d): Conjugate pad
- (e): Sample pad
- (f): Absorption pad
- (g): Third pad
- (h): Conjugate region
- (i): Film

## Claims

1. An immunochromatographic assay method for an analyte, the method comprising using a test strip comprising following (1) to (3):
(1) a sample pad containing a sample application region for applying a biological sample that may contain the analyte;
(2) an insoluble membrane containing at least one detection region to which a binding partner immunologically reactive with the analyte is immobilized; and
(3) a conjugate containing a binding partner immunologically reactive with the analyte, the binding partner being immobilized to a label;
**characterized in that** the conjugate is retained in a conjugate immobilization region together with an anionic surfactant.

2. The immunochromatographic assay method according to claim 1, wherein the analyte is a respiratory tract virus.

3. The immunochromatographic assay method according to claim 1 or 2, wherein the binding partner is a monoclonal antibody.

4. The immunochromatographic assay method according to any one of claims 1 to 3, **characterized in that** the conjugate immobilization region is provided, as a conjugate region between the sample application region and the detection region in the (1) or (2).

5. The immunochromatographic assay method according to claim 4, wherein a concentration of the anionic surfactant is 0.1 to 20% by mass in terms of a concentration in a conjugate application solution for application to the conjugate region in preparation of the test strip.

6. The immunochromatographic assay method according to any one of claims 1 to 5, wherein the biological sample is respiratory secretions.

7. The immunochromatographic assay method according to any one of claims 1 to 6, wherein the respiratory tract virus is influenza B virus.

8. The immunochromatographic assay method according to any one of claims 1 to 7, wherein the anionic surfactant is a sodium polyoxyethylene alkyl ether sulfate-based anionic surfactant.

9. An immunochromatographic test strip comprising the following (1) to (3):
(1) a sample pad containing a sample application region for applying a biological sample that may contain an analyte;
(2) an insoluble membrane containing at least one detection region to which a binding partner immunologically reactive with the analyte is immobilized; and
(3) a conjugate immobilization region containing the conjugate together with an anionic surfactant.

10. The immunochromatographic test strip according to claim 9, wherein the analyte is a respiratory tract virus.

11. The immunochromatographic test strip according to claim 9 or 10, wherein the binding partner is a monoclonal antibody.

12. The immunochromatographic test strip according to any one of claims 9 to 11, **characterized in that** the conjugate immobilization region is provided, as a conjugate region between the sample application region and the detection region in the (1) or (2).

13. The immunochromatographic test strip according to any one of claims 9 to 12, wherein a concentration of the anionic surfactant is 0.1 to 20% by mass in terms of a concentration in a conjugate application solution for application to the conjugate region in preparation of the test strip.

14. The immunochromatographic test strip according to any one of claims 9 to 13, wherein the biological sample is respiratory secretions.

15. The immunochromatographic test strip according to any one of claims 9 to 14, wherein the respiratory tract virus is influenza B virus.

16. The immunochromatographic test strip according to any one of claims 9 to 15, wherein the anionic surfactant is a sodium polyoxyethylene alkyl ether sulfate-based anionic surfactant.

17. A method of suppressing non-specific reaction in an immunochromatographic assay method for an analyte, the method comprising using a test strip comprising the following (1) to (3) :
(1) a sample pad containing a sample application region for applying a biological sample that may contain the analyte;
(2) an insoluble membrane containing at least one detection region to which a binding partner immunologically reactive with the analyte is immobilized; and
(3) a conjugate immobilization region containing a conjugate together with an anionic surfactant.

18. The method of suppressing non-specific reaction according to claim 17, wherein the analyte is a respiratory tract virus.
